# EUROPEAN PATENT APPLICATION

(11) **EP 2 145 582 A1**
(43) Date of publication of application: **20.01.2010**
(21) Application number: 09164640.6
(22) Date of filing: 06.07.2009
(51) Int. Cl.: A61B 8/00, G01S 7/52

(54) **Ultrasound system and method for operating the same**

(30) Priority: 15.07.2008 KR 20080068673
(71) Applicant: MEDISON CO., LTD., Kangwon-do 250-870 (KR)
(72) Inventor: Hyun, Dong Gyu, Gwangju-si Gyeonggi-do (KR)
(74) Representative: Schmid, Wolfgang

(57) **Abstract**

An ultrasound system capable of displaying and manipulating an ultrasound image and an exterior image, and a method for operating the same are disclosed. The system includes an ultrasound diagnosis unit (10) including a probe which sends an ultrasound signal to a target and receives a reflected ultrasound signal from the target, an exterior image storage (20) which stores exterior image data, an image processor (50) which forms an ultrasound image and an exterior image, a user input unit (40) operated to manipulate and display the reflected ultrasound signal and the exterior image data, a central processor (30) which controls the ultrasound diagnosis unit (10) and the exterior image storage (20) to allow the image processor to manipulate and form the ultrasound and exterior images, and a display unit (60) which displays the ultrasound and exterior images, a manipulated state of the ultrasound and exterior images, and an operating state of the central processor (30).

## Description

### BACKGROUND OF THE INVENTION

### 1. Field of the Invention

The present invention relates to ultrasound systems and, more particularly, to an ultrasound system that permits not only simultaneous display of an ultrasound image and exterior images, but also manipulation of image data of the exterior images in a desired form, and to a method for operating the same.

### 2. Description of the Related Art

With development of electronic instruments, a variety of medical instruments based on various principles have also been developed to observe the internal organs of a patient body. For example, an operator can observe the internal organs of the body in a non-destructive and non-invasive manner with ultrasound waves via an ultrasound system.

The ultrasound system facilitates easier acquisition of images in real time than medical imaging modalities such as a computerized tomography (CT), a magnetic resonance imager (MRI), a positron emission tomography (PET), etc., and does not entail any danger of radiation exposure, which can occur in CT and the like.

Here, it should be noted that the aforementioned description is not a description of a conventional technique, but a description of the pertinent art to which the present invention pertains.

An operator can obtain the internal images of a patient body in real time using the ultrasound system without causing any damage to the body. However, the images from the ultrasound system have a lower resolution or definition than the images from the CT, MRI, PET and the like, making it difficult to clearly discriminate a lesion at a precise location.

In the case where it is difficult to discriminate the lesion with the images obtained via the ultrasound system, the operator reads out and discriminates the lesion from a separate PACS (Picture Archiving and Communication System) workstation by observing CT or MRI images of the patient during ultrasound examination.

As such, when the conventional ultrasound system is also installed in a single ultrasound scan room, not only does spatial loss of the ultrasound scan room occur, but work efficiency is also deteriorated in the case of operating the PACS workstation to observe the CT or MRI images during the ultrasound examination.

### SUMMARY OF THE INVENTION

The present invention is conceived to solve the problems of the prior art as described above, and an aspect of the present invention is to provide an ultrasound system that permits not only simultaneous display of ultrasound images and exterior images, but also manipulation of image data of the exterior images in a desired form. Another aspect of the present invention is to provide a method for operating the ultrasound system.

In accordance with an aspect of the present invention, an ultrasound system capable of displaying and manipulating an ultrasound image and an exterior image is provided. The ultrasound system includes: an ultrasound diagnosis unit including a probe configured to send an ultrasound signal to a target and to receive a reflected ultrasound signal from the target; an exterior image storage configured to store exterior image data sent from an exterior imaging modality; an image processor configured to form an ultrasound image based on the reflected ultrasound signal sent from the ultrasound diagnosis unit and to form an exterior image based on the exterior image data sent from the exterior image storage; a user input unit operated to manipulate and display the reflected ultrasound signal and the exterior image data; a central processor configured to control the ultrasound diagnosis unit and the exterior image storage according to operation of the user input unit to allow the image processor to manipulate and form the ultrasound image and the exterior image; and a display unit configured to display the ultrasound image and the exterior image formed by the image processor, a manipulated state of the ultrasound image and the exterior image, and an operating state of the central processor.

The exterior image data may include images obtained via CT, MRI, SPECT (Single Photon Emission Computerized Tomography), X-ray, flouroscopy, and endoscopy.

The exterior image data may be multislice data.

The exterior image data may be stored via classification according to IDs of patients.

The exterior image data may be image data of DICOM (Digital Imaging Communication in Medicine) format.

The exterior image storage may include a PACS server, a portable storage, and a hard disc.

The reflected ultrasound signal may be input in real time to the image processor.

The user input unit may include a key panel, numeric keys, a keyboard, a foot switch, a remote controller, a track ball, and a mouse.

In accordance with another aspect of the present invention, a method for operating an ultrasound system capable of displaying and manipulating an ultrasound image and an exterior image is provided. The method includes: inputting an ID of a patient with a user input unit; retrieving exterior image data corresponding to the ID of the patient; arranging the retrieved exterior image data on a screen; displaying exterior image data selected among the arranged exterior image data in a preset mode on the screen; and manipulating the displayed exterior image data.

The exterior image data may include images obtained via CT, MRI, SPECT, X-ray, flouroscopy, and endoscopy.

The exterior image data may be multislice data.

The exterior image data may be stored via classification according to IDs of patients.

The user input unit may include a key panel, numeric keys, a keyboard, a foot switch, a remote controller, a track ball, and a mouse.

The preset mode may be one of a mode of displaying one of the selected image data as a thumbnail at a lower end of the screen and a mode of displaying one of the selected image data on the whole screen while permitting screen conversion via a tab at the lower end of the screen.

The manipulating step may comprise one of regulating a position of a slice displaying multislice data, regulating brightness and contrast, changing an arrangement of the exterior image data, changing a view direction, and converting a main/subordinate relation between the exterior image data and the ultrasound image or changing an input focus.

### BRIEF DESCRIPTION OF THE DRAWINGS

The above and other features and advantages of the present invention will become apparent from the following description of exemplary embodiments given in conjunction with the accompanying drawings, in which:
Fig. 1 is a block diagram of an ultrasound system according to one embodiment of the present invention;
Figs. 2 to 5 are images displayed on a screen of the ultrasound system according to the embodiment of the present invention;
Fig. 6 is a schematic view of an ultrasound system according to one embodiment of the present invention; and
Fig. 7 is a flowchart of a method for operating an ultrasound system according to one embodiment of the present invention.

### DETAILED DESCRIPTION OF THE INVENTION

Exemplary embodiments of the present invention will be described in detail with reference to the accompanying drawings hereinafter. It should be noted that the drawings are not to precise scale and may be exaggerated in thickness of lines or size of components for descriptive convenience and clarity only. Furthermore, terms used herein are defined by taking functions of the present invention into account and can be changed according to the custom or intention of users or operators. Therefore, definition of the terms should be made according to overall disclosures set forth herein.

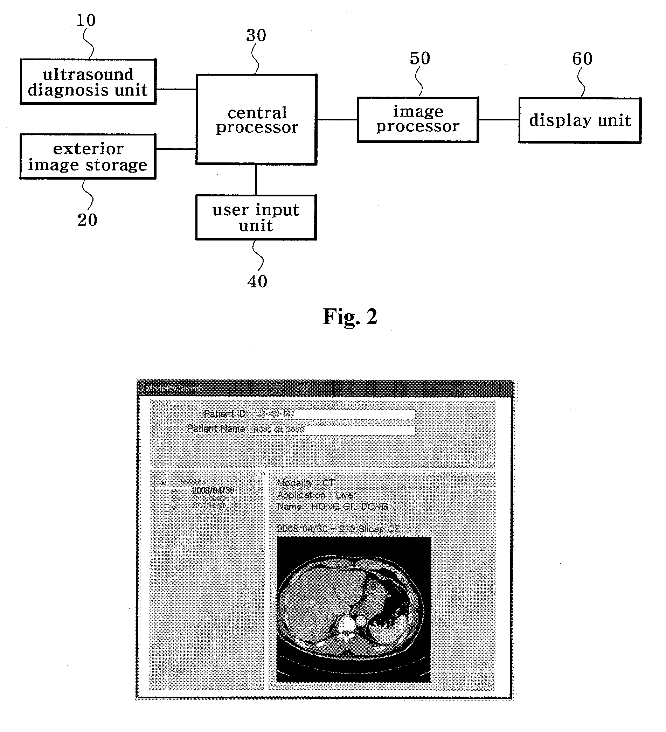

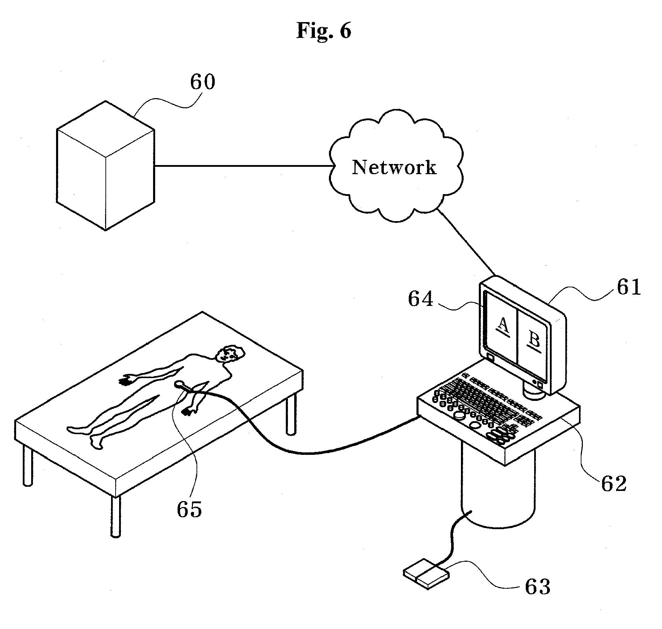

Fig. 1 is a block diagram of an ultrasound system according to one embodiment of the invention, Figs. 2 to 5 are images displayed on a screen of the ultrasound system according to the embodiment, and Fig. 6 is a schematic view of the ultrasound system according to the embodiment.

Referring to Fig. 1, the ultrasound system according to one embodiment of the invention includes an ultrasound diagnosis unit 10, an exterior image storage 20, an image processor 50, a user input unit 40, a central processor 30, and a display unit 60.

For ultrasound diagnosis according to this embodiment, the ultrasound diagnosis unit 10 sends an ultrasound signal to a target (not shown) and receives a reflected ultrasound signal from the target through a probe (not shown).

The exterior image storage 20 serves to store exterior image data sent from an exterior imaging modality (not shown). As the exterior image storage 20, any of devices such as a server of a picture archiving and communication system (PACS), USB type portable storages, and hard discs can be used, so long as the storage acts as a means for storing the exterior images and can be connected to the ultrasound system.

Here, the exterior image data is image data of DICOM (Digital Imaging Communication in Medicine) format, which is a standard format of international medical images. The exterior image data may include images obtained via CT, MRI, SPECT, X-ray, flouroscopy and endoscopy, and may be constituted by multislice images.

Further, the exterior image data are stored after classification based on IDs of patients as shown in Fig. 2, so that the exterior images can be searched from the exterior image storage 20 such as PACS and the like when an operator wishes to check the exterior images on the ultrasound system. As such, the exterior images are stored by date according to the IDs of patients, such that CT image data taken on a certain date is read out and displayed on a screen of the system when a certain date is chosen.

The central processor 30 controls the ultrasound diagnosis unit 10 and the exterior image storage 20 according to operation of the user input unit 40 to allow the image processor 50 to manipulate and form an ultrasound image and an exterior image. The image processor 50 forms the ultrasound image based on the reflected ultrasound signal sent in real time from the ultrasound diagnosis unit 10, and forms the exterior image based on the exterior image data sent from the exterior image storage 20.

The user input unit 40 is used to manipulate and display the reflected ultrasound signal and the exterior image data. The user input unit 40 includes a key panel, numeric keys, a keyboard, a foot switch, a remote controller, a track ball, and a mouse.

The user input unit 40 is used not only for manipulation of the ultrasound system, but also for display and manipulation of the exterior image data.

Table 1 shows one example of a mapped operation for manipulating CT image data among exterior image data with the key panel and foot switch of the ultrasound system.

**Table 1**

| Key panel | Ultrasound system | CT image data manipulation |
|---|---|---|
| Trackball | CINE pos, indicator, annotation, cursor | Slice change (Prev/Next) |
| B Gain | B mode Gain | Brightness |
| C Gain | C mode Gain | Contrast |
| Single/Dual/Quad | Single/Dual/Quad layout | Layout (1x1, 2x1, 2x2) |
| X, Y, Z | 3D X, Y, Z | Axial, Coronal, Sagital |
| Change | Trackball change | US/CT Focus Change |
| 3D | 3D/4D US | CT 3D Reconstruction |
| Foot switch | Ultrasound system | CT image data manipulation |
| Left | Freeze | US/CT Image Change |
| Right | Print | Slice Change (Prev/Next) |

The display unit 60 displays the ultrasound image and the exterior image formed by the image processor 50, and an operating state of the central processor 30.

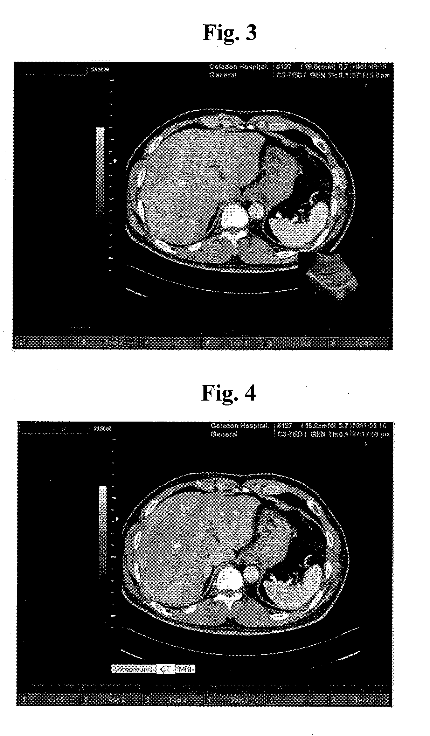

As shown in Fig. 3, the image processor 50 can read out the multislice CT image data from the exterior image storage 20 to display CT images on the whole screen of the display unit 60 while displaying an ultrasound image at a right side lower end of the screen. When the system is set to receive the CT image data as input data, the operator can adjust the CT image data with the key panel and the foot switch.

In Fig. 4, a screen which displays several exterior images and an ultrasound image according to one embodiment of the invention is illustrated. As described above, in one embodiment of the invention, CT images and MRI images are simultaneously read out and displayed along with the ultrasound image. At this time, unselected exterior images are displayed as icons at the lower end of the screen, while a selected exterior image is displayed on the whole screen. The ultrasound image is operated as a background in real time, and can be displayed on the whole screen when selected by the operator.

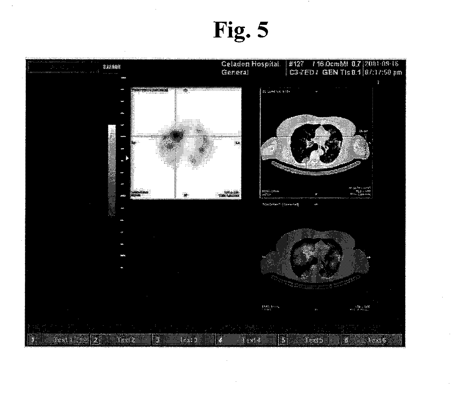

Fig. 5 shows a screen which displays a multiplanar view of SPECT data provided as the exterior image data in the ultrasound system according to the embodiment of the invention.

Fig. 6 is a schematic view of an ultrasound system according to one embodiment of the present invention.

Referring to Fig. 6, an operator scans a patient with the probe 65, and manipulates an ultrasound image, displayed on a display screen 64 of the ultrasound image unit 61, with the key panel 62 and the foot switch 63 to perform ultrasound diagnosis of the patient. When an exterior image A is needed during the ultrasound diagnosis, the operator manipulates the key panel 62 and the foot switch 63 to display the exterior image A such as a CT image on the display screen 64. As described above, the exterior image A and the ultrasound image B may be simultaneously or alternately displayed as needed. Other exterior image data in different formats and CT images stored in a PACS server 66 are sent to an ultrasound imaging unit 61 via a network.

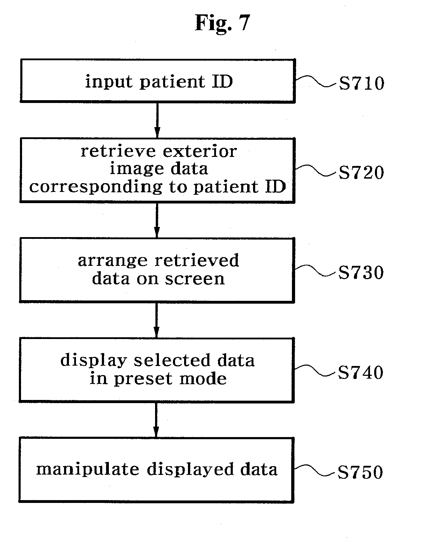

Fig. 7 is a flowchart of a method for operating the ultrasound system according to one embodiment of the present invention.

Referring to Figs. 1 and 7, when an ID of a patent is input via the user input unit 40 in operation S710, the central processor 30 retrieves exterior image data corresponding to the ID of the patient in operation S720.

The exterior image data retrieved by the central processor 30 is arranged on the display unit 60 through the image processor 50 in operation S730.

The display unit 60 allows the exterior image data selected by the operator to be displayed in a selected preset mode on the screen of the display unit in operation S740. Then, in operation S750, the operator manipulates the exterior image data while changing the exterior image data or the mode for displaying the image data, as needed.

Here, the preset mode may be one of a mode of displaying one of the selected image data as a thumbnail at the lower end of the screen and a mode of displaying one of the selected image data on the whole screen while permitting screen conversion via a tab at the lower end of the screen.

Further, the manipulation of the exterior image data by the operator may comprise one of regulating a position of a slice which displays multislice data, regulating brightness and contrast, changing an arrangement of the exterior image data, changing a view direction, and converting a main/subordinate relation between the exterior image data and the ultrasound image or changing an input focus.

As such, the ultrasound system allows the operator to check the exterior images during the ultrasound diagnosis by manipulating the exterior image data while viewing the exterior image data, thereby improving operation efficiency while ensuring accurate ultrasound diagnosis.

As apparent from the above description, the present invention enables not only simultaneous display of ultrasound images and exterior images, but also manipulation of image data of the exterior images to be displayed in a desired form, so that the exterior images can be observed with the single ultrasound system during ultrasound diagnosis, thereby improving diagnosis efficiency while ensuring accurate ultrasound diagnosis.

Further, the present invention eliminates requirement for a separate instrument for checking exterior images, thereby improving spatial usage while releasing operator burdens from operation of the separate instrument.

Although the present invention has been described with reference to the embodiments and the accompanying drawings, it will be apparent to those skilled in the art that the embodiments are given by way of illustration, and that various modifications and equivalent embodiments can be made without departing from the spirit and scope of the present invention. Accordingly, the scope of the present invention should be limited only by the accompanying claims.

## Claims

1. An ultrasound system capable of displaying and manipulating an ultrasound image and an exterior image, comprising:
an ultrasound diagnosis unit (10) including a probe configured to send an ultrasound signal to a target and to receive a reflected ultrasound signal from the target;
an exterior image storage (20) configured to store exterior image data sent from an exterior imaging modality;
an image processor (50) configured to form an ultrasound image based on the reflected ultrasound signal sent from the ultrasound diagnosis unit (10) and to form an exterior image based on the exterior image data sent from the exterior image storage (20);
a user input unit (40) operated to manipulate and display the reflected ultrasound signal and the exterior image data;
a central processor (30) configured to control the ultrasound diagnosis unit (10) and the exterior image storage (20) according to operation of the user input unit to allow the image processor (50) to manipulate and form the ultrasound image and the exterior image; and
a display unit (60) configured to display the ultrasound image and the exterior image formed by the image processor (50), a manipulated state of the ultrasound image and the exterior image, and an operating state of the central processor (30).

2. The ultrasound system according to claim 1, wherein the exterior image data comprises images obtained via CT, MRI, SPECT, X-ray, flouroscopy, and endoscopy.

3. The ultrasound system according to claim 1, wherein the exterior image data is multislice data.

4. The ultrasound system according to claim 1, wherein the exterior image data is stored via classification according to IDs of patients.

5. The ultrasound system according to any one of claims 1 to 4, wherein the exterior image data is image data of DICOM format.

6. The ultrasound system according to claim 1, wherein the exterior image storage comprises a PACS server, a portable storage, and a hard disc.

7. The ultrasound system according to claim 1, wherein the reflected ultrasound signal is input in real time to the image processor (50).

8. The ultrasound system according to claim 1, wherein the user input unit (40) comprises a key panel, numeric keys, a keyboard, a foot switch, a remote controller, a track ball, and a mouse.

9. A method for operating an ultrasound system capable of displaying and manipulating an ultrasound image and an exterior image, the method comprising:
inputting an ID of a patient with a user input unit (40);
retrieving exterior image data corresponding to the ID of the patient;
arranging the retrieved exterior image data on a screen;
displaying exterior image data selected among the arranged exterior image data in a preset mode on the screen; and
manipulating the displayed exterior image data.

10. The method according to claim 9, wherein the exterior image data comprises images obtained via CT, MRI, SPECT, X-ray, flouroscopy, and endoscopy.

11. The method according to claim 9, wherein the exterior image data is multislice data.

12. The method according to claim 9, wherein the exterior image data is stored via classification according to IDs of patients.

13. The method according to claim 9, wherein the user input unit (40) comprises a key panel, numeric keys, a keyboard, a foot switch, a remote controller, a track ball, and a mouse.

14. The method according to claim 9, wherein the preset mode is one of a mode of displaying one of the selected image data as a thumbnail at a lower end of the screen and a mode of displaying one of the selected image data on the whole screen while permitting screen conversion via a tab at the lower end of the screen.

15. The method according to claim 9, wherein the manipulating step comprises one of regulating a position of a slice displaying multislice data, regulating brightness and contrast, changing an arrangement of the exterior image data, changing a view direction, converting a main/subordinate relation between the exterior image data and the ultrasound image, and changing an input focus.
